# EUROPÄISCHE PATENTANMELDUNG

(11) **EP 0 564 932 A1**
(43) Veröffentlichungstag der Anmeldung: **13.10.1993**
(21) Anmeldenummer: 93105085.0
(22) Anmeldetag: 27.03.1993
(51) Int. Cl.: C07D 285/12, C09K 19/58, C09K 19/34

(54) **Verwendung der chiralen Gruppe (1S,4R)-1,4-Dihydroxy-2-cyclopentenyl zur Herstellung von polaren, flüssigkristallinen Materialien**

(30) Priorität: 08.04.1992 DE 4211692
(71) Anmelder: BASF Aktiengesellschaft, D-67063 Ludwigshafen (DE)
(72) Erfinder: Siemensmeyer, Karl, Dr., W-6710 Frankenthal (DE); Tschierske, Carsten, Dr., O-4070 Halle/Saale (DE); Joachimi, Detlev, O-4059 Halle/Saate (DE); Theil, Fritz, Pr. Dr., O-1055 Berlin (DE)

(57) **Zusammenfassung**

Verwendung der chiralen Gruppe mit der allgemeinen Formel 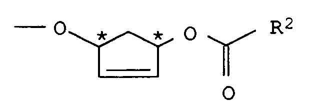
als Bestandteil flüssigkristalliner Materialien.

## Beschreibung

Die vorliegende Erfindung betrifft die Verwendung von (1S, 4R) -1, 4-Dihydroxy-2-cyclopentenyl zur Herstellung von polaren, flüssigkristallinen Materialien mit der folgenden allgemeinen Struktur:

P-A-B-C I

in der die Variablen folgende Bedeutung haben:
- P:: Polymerisierbare Einheit oder H oder OH oder Ethenyl;
- A:: flexibler, abstandhaltender Molekülteil;
- B:: aus mindestens zwei linear oder angenähert linear miteinander verknüpften aromatischen Kernen aufgebauter Molekülteil;
- C:: optisch aktiver, polarer und chiraler Molekülteil mit der folgenden allgemeinen Struktur:

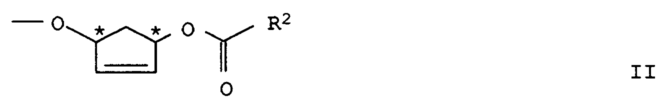

wobei R² eine lineare oder verzweigte, chirale oder achirale Alkylgruppe sein kann, die auch durch Cl, Br, F, CN oder OH substituiert sein kann.

Weiterhin betrifft die Erfindung die Herstellung von Polymeren aus den Monomeren der allgemeinen Formel I wenn P die folgende Gruppe ist: 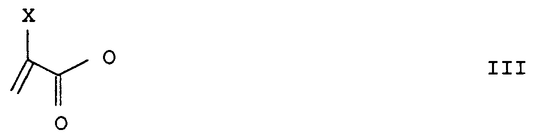
in der X gleich Methyl, Chlor, Brom, CN oder H sein kann, sowie deren Verwendung zum Aufbau von Aufzeichnungsschichten für laseroptische und elektrische Aufzeichnungselemente sowie in der Elektrophotographie, zur Erzeugung latenter Ladungsbilder und zum Aufbau oder als Bestandteil von flüssigkristallinen Anzeigeelementen.

Chiral, smektisch, flüssigkristalline Materialien, die beim Abkühlen aus der flüssigkristallinen Phase glasartig unter Ausbildung einer Schichtstruktur erstarren, werden bekanntermaßen auf elektrooptischem Gebiet für viele Zwecke eingesetzt. Zu nennen sind hier beispielsweise optische Speichersysteme (DE-A-38 27 603 und DE-A-39 17 196), die Elektrophotographie (DE-A-39 30 667), flüssigkristalline Anzeigeelemente wie Displays (Mol. Cryst. Liq. Cryst. 114,151-187, (1990)) sowie bei gleichzeitig vorliegendem ferroelektrischen Verhalten elektrische Speichersysteme (Ferroelectrics, 104,241-256, (1990)).

In der Schichtstruktur ferroelektrischer S_{c}*-Phasen sind die Moleküllängsachsen innerhalb der einzelnen Schicht gegenüber der Schichtnormale z geneigt. Die Richtung dieser Neigung wird durch den Direktor n angegeben, der Winkel zwischen z und n ist der sogenannte Tiltwinkel Θ. S_{c}*-Phasen weisen zwei stabile Zustände mit unterschiedlicher Richtung von n auf, zwischen denen durch Anlegen eines elektrischen Feldes schnell geschaltet werden kann (elektrooptischer Effekt).

S_{c}*-Phasen treten bei niedermolekularen, flüssigkristallinen Materialien und auch bei polymer flüssigkristallinen Materialien auf, wobei die wesentlichen Eigenschaften der S_{c}*-Phase übereinstimmen.

Die bislang hergestellten flüssigkristallinen Materialien weisen jedoch Nachteile auf wie zum Beispiel geringe spontane Polarisation, geringe Phasenbreiten, kein stabiles, getiltet smektisches Glas bei Raumtemperatur, oder hohe Schaltzeiten.

Das Auftreten der flüssigkristallinen S_{c}*-Phasen wird durch alle Baugruppen des Materials, Spacer^{A}, Mesogen^{M} und chiraler^{C}-Gruppen in erheblichem Ausmaß beeinflußt, so daß kleinste Änderungen der molekularen Struktur S_{c}*-Phasen induzieren oder auch zum Verschwinden bringen können.

Speziell die chirale Gruppe ist durch ihre Struktur und spezielle Funktion für das Zustandekommen einer spontanen Polarisation von entscheidender Bedeutung.

Der vorliegenden Erfindung lag daher die Aufgabe zugrunde, chirale Bauelemente für flüssigkristalline Materialien zu suchen, die die flüssigkristallinen Eigenschaften möglichst wenig stören und gleichzeitig hohe, spontane Polarisation induzieren.

Diese Aufgabe wurde erfindungsgemäß durch die Verwendung von (1S,4R)-1,4-Dihydroxy-2-cyclopentenyl als chirale Gruppierung gelöst.

Die zugrundeliegenden flüssigkristallinen Vorstufen mit der allgemeinen Struktur

P-A-B- IV,

in der P, A, B die oben angegebene Bedeutung haben, sind aus der Literatur, z.B. DE-A-39 17 196, bekannt.

Bei dem Molekülteil P handelt es sich, bei niedermolekularen ferroelektrischen Materialien vorzugsweise um Wasserstoff. Zur Anbindung an Polysiloxan oder Polyacrylsäure-Ketten im Sinne einer polymeranalogen Umsetzung wird vorzugsweise für P eine Hydroxylgruppe oder eine Ethenylgruppe bevorzugt. Hingegen zur Herstellung von radikalisch oder anionisch zu polymerisierenden Materialien wird eine in α-Position mit H, Cl, Br, CN oder Methyl substituierte Acrylsäure bevorzugt.

Bei dem Molekülteil A handelt es sich vorzugsweise um eine Alkylengruppe -(CH₂)ₙ- mit n = 2 bis 20, bevorzugt mit n = 6 bis 11, wobei jede dritte CH₂-Gruppe durch O (Sauerstoff) oder S (Schwefel) oder NH ersetzt sein kann.

Der mesogene Molekülteil B ist aus mindestens zwei linear oder angenähert linear miteinander verknüpften aromatischen Kernen aufgebaut.

Bevorzugte Gruppierungen B sind beispielsweise solche der Formeln V bis IX: 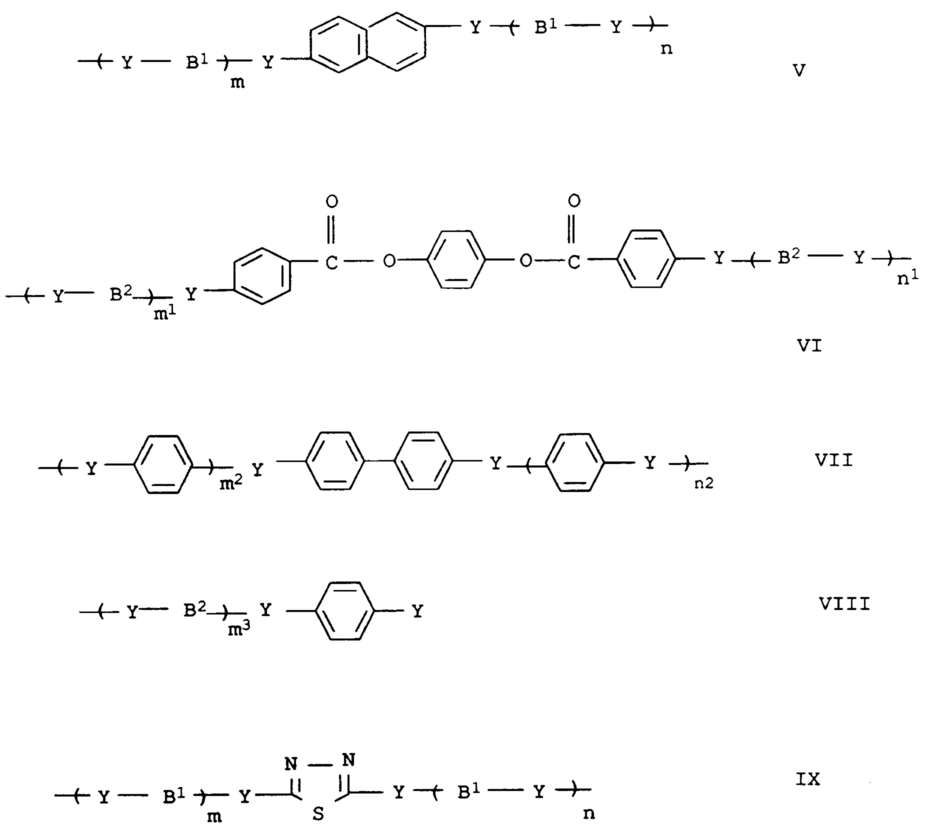
wobei die Variablen folgende Bedeutung haben:
- B¹: gleiche oder verschiedene der folgenden Reste: p-Phenylen oder Biphen-4,4'-ylen;
- B²: gleiche oder verschiedene der folgenden Reste: p-Phenylen, Biphen-4,4'-ylen, Naphth-2,6-ylen, 1,3,4-Thiadiazolen oder Pyrimidylen;
- Y: gleiche oder verschiedene der folgenden Gruppen: bevorzugt -O-, -COO-, -OCO- oder eine chemische Bindung daneben auch -CH₂-O-, -O-CH₂-, -COS- oder -SCO-.
- m,n: 0,1 oder 2, wobei m und n nicht gleichzeitig 0 bedeuten dürfen
- m¹,n¹: unabhängig voneinander 0, 1 oder 2
- m²,n²: unabhängig voneinander 0 oder 1
- n³: 1 oder 2
Beispiele für besonders bevorzugte Gruppen sind folgende:
Zu V 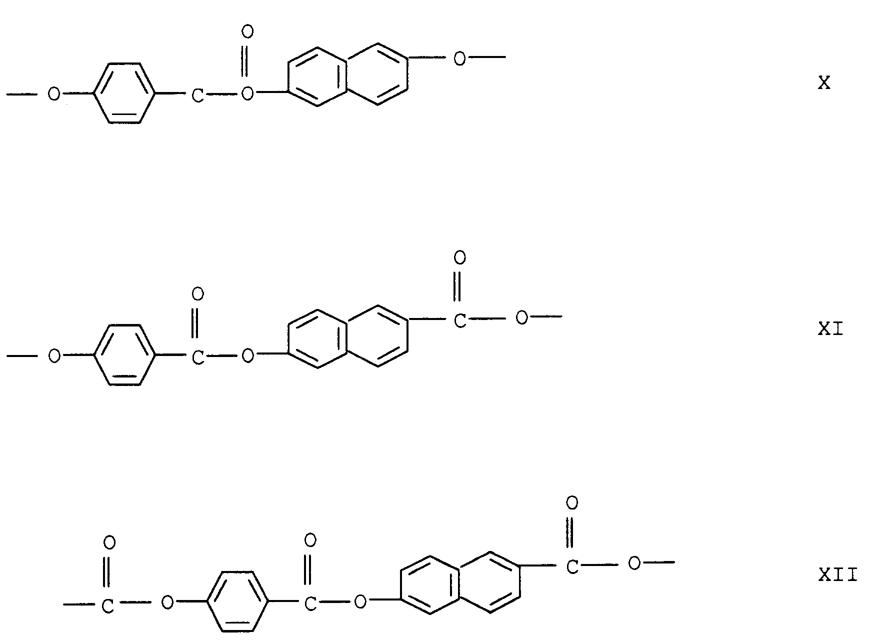 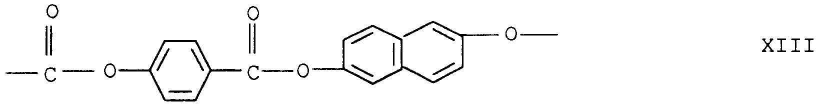
Zu VI 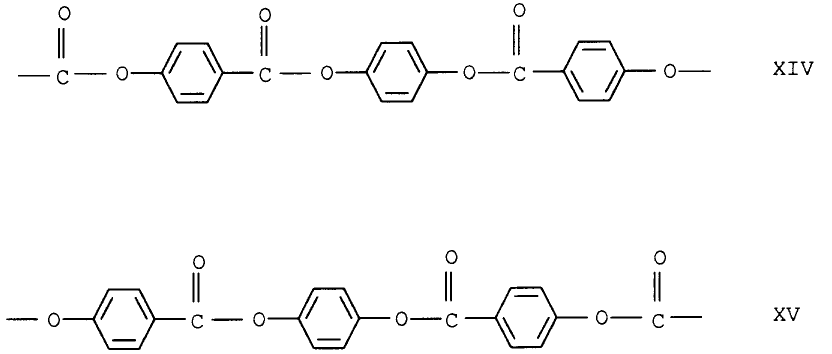
Zu VII 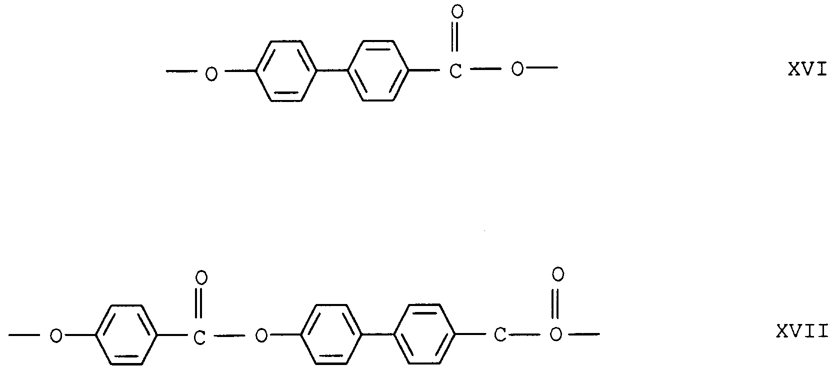 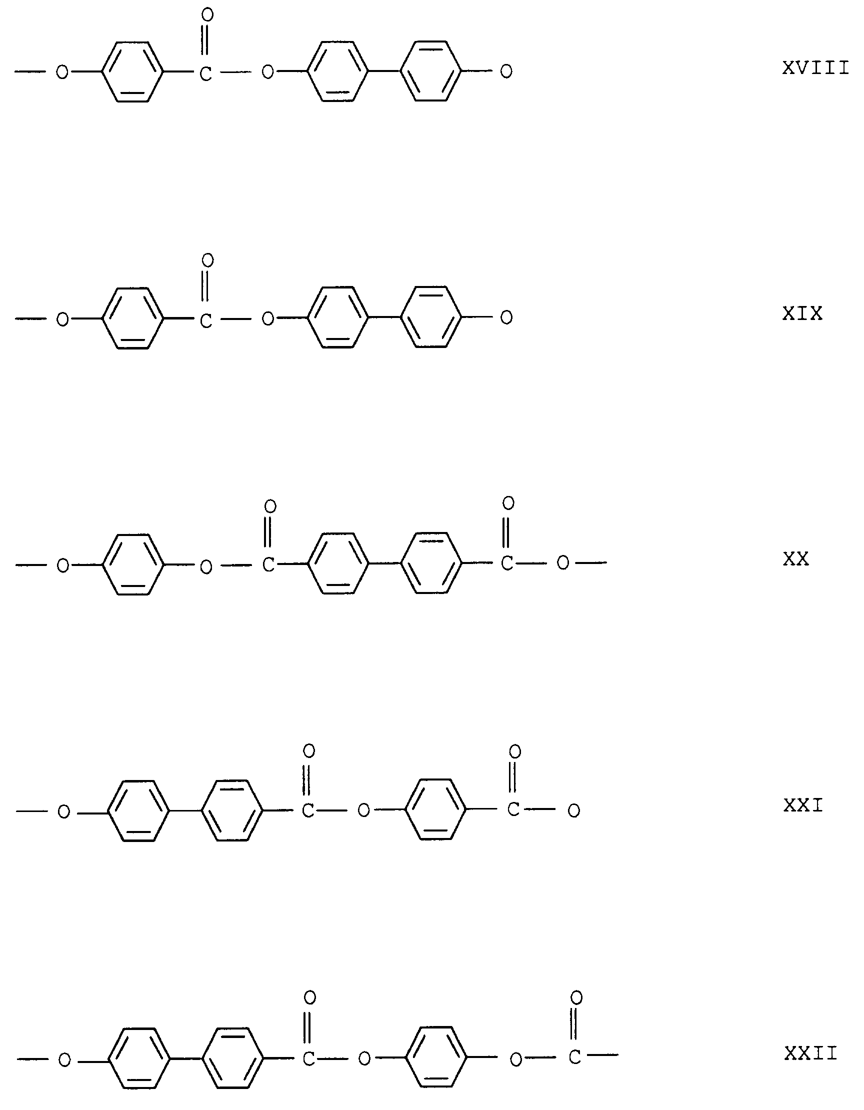 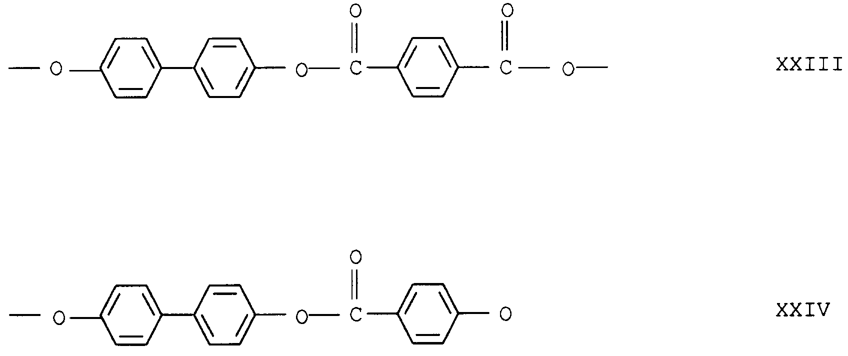
Zu VIII 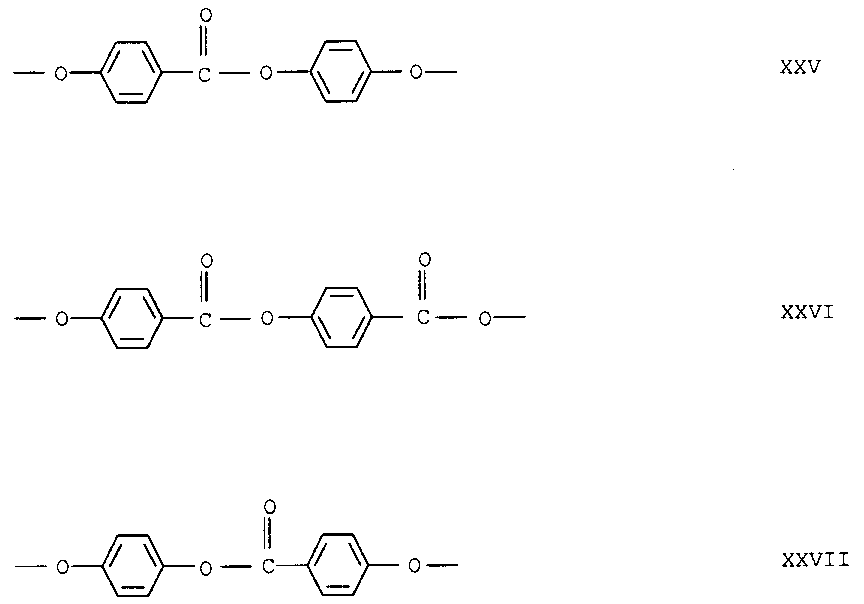 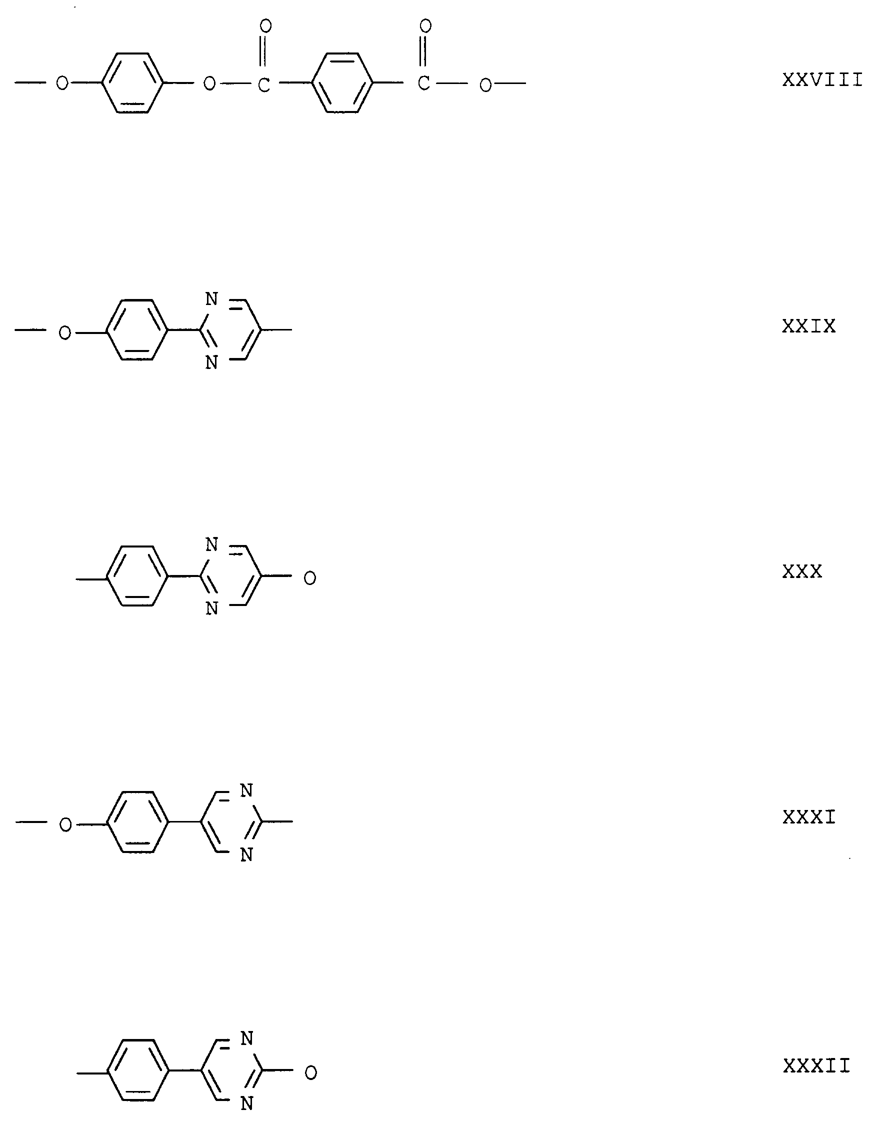
Zu IX 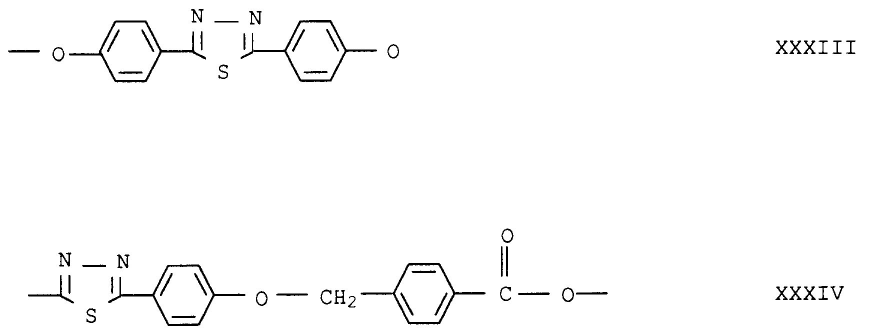
Der chirale Molekülteil C besitzt vorzugsweise folgende Struktur: 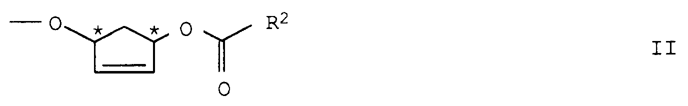
wobei die Variable R² die folgende Bedeutung hat:
R²: lineare oder verzweigte, chirale oder nichtchirale Alkylgruppen, die durch Cl, Br, F, CN, Methyl oder OH substituiert sein kann und dessen längste C-Kette mindestens 1, höchstens 12 C-Atome aufweist, wovon jedoch jedes 3. C-Atom durch O, NH oder S substituiert sein kann.

Beispiele für den Rest C sind folgende Gruppen: 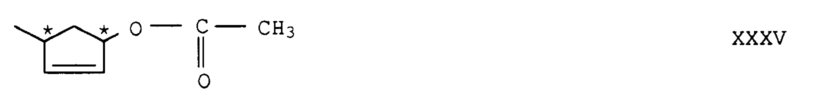 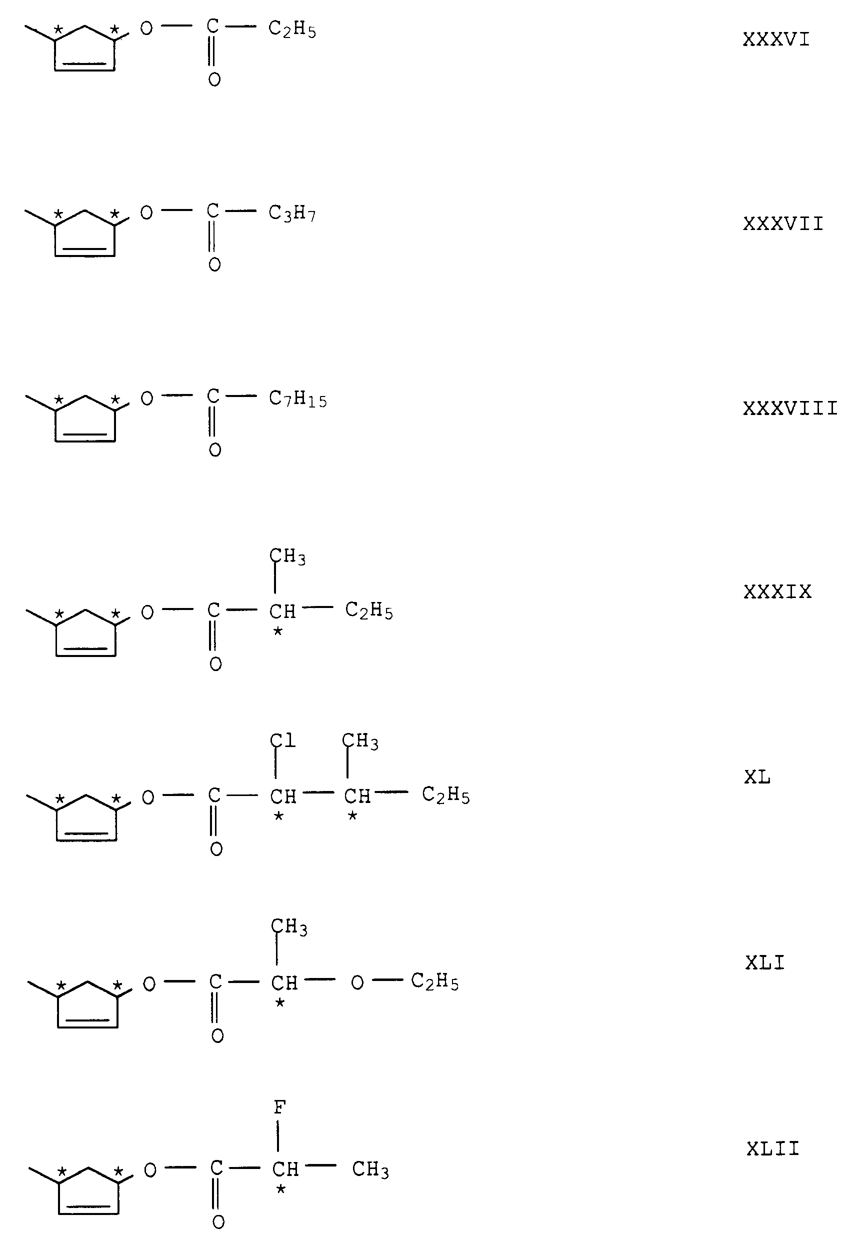
Die erfindungsgemäßen Einheiten P-A-B, in denen P, A, B die oben angegebene Bedeutung besitzen sind im Prinzip durch allgemein bekannte Synthese-Verfahren wie sie in der DE-A-39 17 196 und in Mol. Chryst. Liq. Cryst. 191 (1991), 223 oder Mol. Cryst. Liq. Cryst 191 (1991), 231 beschrieben sind zugänglich.

Die Herstellung von optisch aktivem (1S,4R)-1,4-Dihydroxy-2-cyclopentens wurde erstmals in Angew. Chem. 68 (1956), 248 beschrieben.

Die Phasenumwandlungstemperaturen wurden mit einem Boetius Heiztischmikroskop bestimmt und sind nicht korrigiert.

Die spontane Polarisation wurde mittels einer Brückenmethode nach Diamant et al. bestimmt (Rev.Sci.Instr. 28 (1957), 30.

Allgemeine Arbeitsvorschrift zur Herstellung von (1S,4R)-2-(4-Octyloxyphenyl)-5-[4-carboxy-2-cyclopentenyloxy)-phenyl]1,3,4-thiadiazol durch Mitsunobu Veretherung.

In einem 50 ml Kolben mit CaCl₂-Rohr und Septum werden 3,0 mmol des substituierten Phenols mit 4,5 mmol Triphenylphosphin (1,2 g) und 4,5 mmol des Cycolpentenols in 25 ml abs. THF vorgelegt. Unter Rühren werden bei 0°C bis 5°C 4,5 mmol (0,75 ml) Diethylazodicarboxylat während 10 min zugetropft. Anschließend läßt man 24 h bei Raumtemperatur nachrühren und destilliert dann das Lösungsmittel weitgehend ab. Zur Entfernung von Triphenylphosphinoxid wird zweimal aus Methanol/Wasser (10/1) umkristallisiert. Nachfolgendes mehrmaliges Umkristallisieren aus Ethanol liefert die reine Zielverbindung.

### Beispiele:

### Beispiel 1

### Herstellung von (1S,4R)-2-(4-Octyloxyphenyl)-5-[4-(1-acetoxy-2-cyclopentenyloxy)-phenyl]-1,3,4-thiadiazol

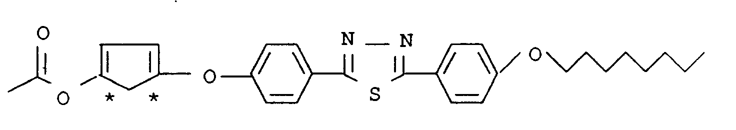

Gemäß der allgemeinen Arbeitsvorschrift wurden eingesetzt
3 mmol 4-Octyloxyphenyl-5-(4-hydroxyphenyl)-1,3,4-thiadiazol (1.15 g)
4,5 mmol (1S,4R)-4-Hydroxy-2-Cyclopentenylacetat (0,63 g)
4,5 mmol Triphenylphosphin (1,2 g)
4,5 mmol Diethylazodicarboxylat (0,75 ml)

### Ausbeute:

0,81 g von (1S,4R)-2- (4-Octyloxyphenyl)-5[4-(1-acetoxy-2-cyclopentenyloxy)-phenyl]-1,3,4-thiadiazol (53 %)

### Phasenverhalten:

K 89,5 S_{c}* 159 N* 181,5 I
Die spontane Polarisation bei 129°C beträgt 86 nC cm⁻²

### Beispiel 2

### Herstellung von: (1S, 4R)-2-(4-Octyloxyphenyl)-5-[4-1-propoxy-2-cyclopentenyloxy)-phenyl]-1,3,4-thiadiazol

### Eingesetzte Mengen:

3 mmol 4-Octyloxyphenyl-5-(4-hydroxyphenyl)-1,3,4-thiadiazol (1.15 g)
4,5 mmol (1S,4R)-4-Hydroxy-2-Cyclopentenylacetat (0,63 g)
4,5 mmol Triphenylphosphin (1,2 g)
4,5 mmol Diethylazodicarboxylat (0,75 ml) 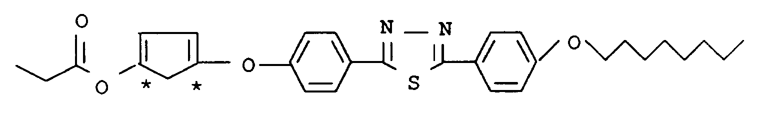
Ausbeute: 0,83 g (55 %)

### Phasenverhalten:

K 92 S_{c} 167 S_{A} 177 I
Die spontane Polarisation bei 137°C beträgt 85 nC cm⁻².

### Beispiel 3

### Herstellung von (1S,4R)-2-(4-Octyloxyphenyl)-5-[4-(1-butoxy-2-cyclopentenyloxy)-phenyl]-1,3,4-thiadiazol

### Eingesetzte Mengen:

3 mmol 4-Octyloxyphenyl-5-(4-hydroxyphenyl)-1,3,4-thiadiazol (1.15 g)
4,5 mmol (1S,4R)-4-Hydroxy-2-Cyclopentenylacetat (0,64 g)
4,5 mmol Triphenylphosphin (1,2 g)
4,5 mmol Diethylazodicarboxylat (0,75 ml) 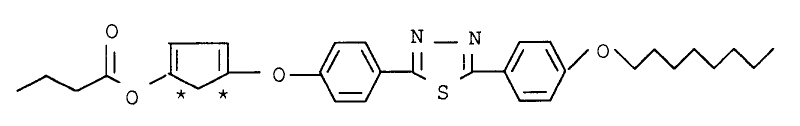
Ausbeute 0,9 g (60 %)

### Phasenverhalten:

K 99 S_{c} 167 N* 172 I
Die spontane Polarisation bei 137°C beträgt 78 nC cm⁻².

### Beispiel 4

### Herstellung von (1S,4R)-2-(4-Octyloxyphenyl)-5-[4-(1-octoxy-2-cyclopentenyloxy)-phenyl]-1,3,4-thiadiazol

### Eingesetzte Mengen:

3 mmol 4-Octyloxyphenyl-5-(4-hydroxyphenyl)-1,3,4-thiadiazol (1.15 g)
4,5 mmol (1S,4R)-4-Hydroxy-2-Cyclopentenyl.....
4,5 mmol Triphenylphosphin (1,2 g)
4,5 mmol Diethylazodicarboxylat (0,75 ml) 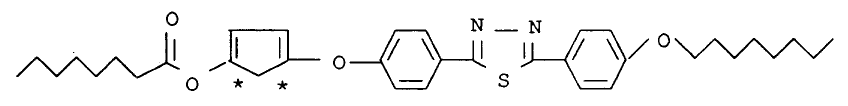
Ausbeute 0,9 g (50 %)

### Phasenverhalten:

K 80.5 Sₓ 91 S_{c}*164.5 N* 167 I
Die spontane Polarisation bei 144°C beträgt 62 nC cm⁻².

### Herstellung von (1S,4R)-2-undecyl-5-[4-(1-acetoxy-2-cyclopentenyloxycarboxybenzylphenyl]-1,3,4-thiadiazol

### Eingesetzte Mengen:

3,1 mmol (1S,4R)-4-Hydroxy-2-Cyclopentenylacetat (Cyclopentenol)
3,1 mmol Dicyclohexylcarbodiimid (DCC)
0,1 mmol Pyrrolidinopyridin (PP)
3,1 mmol 2-undecyl-5-[4-carboxybenzylphenylether]-1,3,4-thiadizol (Carbonsäure) 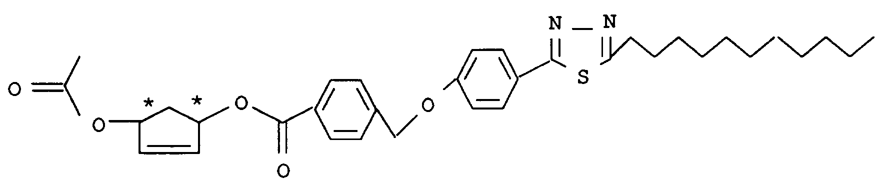
In einem 100 ml Kolben mit CaCl₂-Rohr werden 3,1 mmol des Cyclopentenols, 3,1 mmol DCC und 0,1 mmol PP in CH₂Cl₂ gelöst und unter Eiskühlung bei 0 - 5°C 3,1 mmol der Carbonsäure zugegeben und 24 h bei Raumtemperatur gerührt. Der ausgefallene Harnstoff wird abfiltriert und das Lösungsmittel abgesaugt und das Rohprodukt mehrfach aus Ethanol umkristallisiert.

Ausbeute: 0,83 g (= 60 %)

### Phasenverhalten:

K 103,5 (S_{c}* 80) S_{A} 145 I
Die spontane Polarisation bei 78°C beträgt 8 nC cm-².

## Patentansprüche

1. Substanzen, enthaltend die chirale Gruppe (1S,4R)-1,4-Dihydroxy-2-cyclopenten 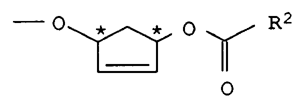 als Bestandteil flüssigkristalliner Materialien.

2. Verwendung von Substanzen gemäß Anspruch 1 als Dotierstoff in flüssigkristallinen Medien.

3. Verwendung der chiralen Gruppe gemäß Anspruch 1 als Baustein flüssigkristalliner niedermolekularer Materialien.

4. Verwendung der chiralen Gruppe gemäß Anspruch 1 als Baustein polymerer flüssigkristalliner Materialien.

5. Verwendung von flüssigkristallinen Medien gemäß Anspruch 1 bis 4 in der Displaytechnologie, in optischen, elektronischen sowie opto-elektronischen Speichermedien, der elektronischen Datenverarbeitung und Vervielfältigung sowie in der Elektrophotographie.
